(19) **Europäisches Patentamt**
**European Patent Office**
**Office européen des brevets**

(11) **EP 1 971 610 B1**

(12) **EUROPEAN PATENT SPECIFICATION**

(45) Date of publication and mention
of the grant of the patent:
**18.11.2009 Bulletin 2009/47**

(51) Int Cl.:
*C07D 495/04* *(2006.01)*    *A61K 31/4365* *(2006.01)*
*A61P 9/00* *(2006.01)*

(21) Application number: **06840999.4**

(22) Date of filing: **15.12.2006**

(86) International application number:
**PCT/EP2006/012129**

(87) International publication number:
**WO 2007/068495 (21.06.2007 Gazette 2007/25)**

(54) **SALTS OF CLOPIDOGREL WITH POLYANIONS AND THEIR USE FOR MANUFACTURING PHARMACEUTICAL FORMULATIONS**

SALZE VON CLOPIDOGREL MIT POLYANIONEN UND IHRE VERWENDUNG ZUR HERSTELLUNG PHARMAZEUTISCHER FORMULIERUNGEN

SELS DE CLOPIDOGREL AVEC POLYANIONS ET UTILISATION POUR LA FABRICATION DE PREPARATIONS PHARMACEUTIQUES

(84) Designated Contracting States:
**AT BE BG CH CY CZ DE DK EE ES FI FR GB GR HU IE IS IT LI LT LU LV MC NL PL PT RO SE SI SK TR**

(30) Priority: **15.12.2005 DE 102005060690**

(43) Date of publication of application:
**24.09.2008 Bulletin 2008/39**

(73) Proprietor: **Capsulution Nanoscience AG**
**12489 Berlin (DE)**

(72) Inventors:
• **DÄHNE, Lars**
**12587 Berlin (DE)**

• **BAUDE, Barbara**
**14548 Schwielowsee (DE)**
• **GONZALEZ FERREIRO, Maria**
**10119 Berlin (DE)**

(74) Representative: **Zimmermann & Partner**
**Isartorplatz 1**
**80331 München (DE)**

(56) References cited:
**EP-A2- 0 191 133**    **EP-A2- 0 211 268**
**WO-A-2004/026879**

**Description**

The present invention relates to salts of the active ingredient clopidogrel, and to methods for preparing them and their use in pharmaceutical formulations.

BACKGROUND OF THE INVENTION

Clopidogrel (methyl (+)-(S)-$\alpha$-(2-chlorophenyl)-6,7-dihydrothieno[3,2-c]pyridine-5(4)-acetate) (Figure 1) is disclosed as active ingredient in EP-A-0 099 802 and US 4 529 596. Clopidogrel acts as inhibitor of platelet aggregation and is therefore preferably employed for preventing thromboembolic events such as, for example, stroke or myocardial infarction.

It is proposed in EP-A-0 281 459 to employ clopidogrel in the form of its inorganic salts, specifically the crystalline hydrogen sulphate. This document also discloses further salts of clopidogrel, but they are described as in some cases amorphous and/or as hygroscopic and are therefore difficult to purify and hard to formulate for pharmaceutical purposes.

According to EP 1 480 985 B1, the clopidogrel hydrogen sulphate predominantly employed in pharmaceutical formulations has, because of the acidic proton, a high acid strength which results in a reduced compatibility with excipients. This patent, as well as WO 2004/072085 A2 and EP 1415993 A1, proposes the use for salt formation of monovalent organic alkyl- and arylsulphonic acids which form substantially crystalline compounds and can readily be purified. These salts are prepared in organic solvents such as dioxane, toluene etc. The-crystallinity desired for purification is particularly pronounced when the salts comprise frequently adsorbed solvent residues as solvate.

The disadvantages of the crystalline clopidogrel hydrogen sulphate and of the crystalline organic alkyl- or arylsulphonates are a limited solubility and slow kinetics of dissolution. US 6,284,277 and WO 2004/026879 therefore disclose preparation of an amorphous formulation of clopidogrel hydrogen sulphate having better solubility than that of the crystalline form by freeze drying the crystalline clopidogrel hydrogen sulphate in the presence of small molecules such as mannitol or alanine, or uncharged polymers (WO 2004/026879) such as polyvinylpyrrolidone, polyethylene glycol, hydroxypropylcellulose. However, the very acidic nature of the hydrogen sulphate remains, thus possibly having adverse effects on the excipients. For example, polyethylene glycol decomposes rapidly under acidic conditions.

EP 191133 discloses salts of S-adenosyl-L-methionine with polyanions.

EP 211268 discloses salts of minoxidil with polyanions

SUMMARY OF THE INVENTION

It has surprisingly been found in our investigations that, contrary to the disclosures in EP-A-0 281 459, WO 2004/072085 A2, EP 1415993 A1, EP 1 480 985 B1, clopidogrel also forms with polyanions stable, substantially amorphous salts which are suitable for manufacturing oral pharmaceutical formulations of the active ingredient. The polyanions can be, for example, carrageenan, polystyrenesulphonate (PSS) or polyvinyl phosphate.

An embodiment of the present invention thus relates to salts of clopidogrel with polyanions. Another embodiment relates to the use of these salts in pharmaceutical formulations. A further embodiment also relates to stable mixed salts which can be precipitated from said clopidogrel-polyanion salts together with monovalent clopidogrel salts such as, for example, hydrochloride. The content of the polyanion salt in these compounds should be at least 10%, preferably at least 20%, more preferably at least 30%, and advantageously more than 40%. A further embodiment of the present invention additionally relates to the process for preparing clopidogrel-polyanion salts, preferably involving a precipitation from acidic aqueous solutions.

Preferably, the polyanion salts of clopidogrel are stable and non-hygroscopic. Further, the salts preferably have a high content of clopidogrel and a low content of additional hydrogen ions. Furthermore, the salts can be prepared on the industrial scale. Moreover, the salts typically dissolve easily and rapidly in the gastrointestinal tract. Particularly for pharmaceutical applications stable and non-hygroscopic polyanion salts of clopidogrel are preferred.

Clopidogrel can be employed both as racemic mixture of the two isomers and as pure isomer, with preference for the (S)-(+) isomer because of the higher activity.

Polyanions are macromolecular compounds having molecular weights preferably above 500 g/mol and at least 4 negative charge units, preferably more than 7 and very particularly preferably having more than 10 charges per molecule. The anionic charges are preferably achieved by acid groups having $pK_a$ values of < 3.5, which are known for example for organic sulphate, sulphonate or phosphate groups and some carboxylate groups. Known polyanions having such properties are on the one hand naturally occurring compounds and modifications thereof, such as, for example, carrageenan, chondroitin sulphate, heparin, dextran sulphate, sulphoethylcellulose, DNA. Synthetic polyanions are additionally suitable, such as, for example, polystyrenesulphonate, polyanetholesulphonate, polyvinyl sulphate, polyvinylsulphonate, polyphosphate, polyvinylphosphonate. Salt formation with heparin, polyanetholesulphonate or with DNA allows combination products to be manufactured, with parenteral formulations (in some circumstances as depot product) being also possible, in contrast to oral uses of clopidogrel to date.

The prepared polysalts are, in contrast to the crystalline salts described to date, are partially amorphous and preferably substantially or predominantly amorphous or completely amorphous, allowing better kinetics of dissolution to be achieved than with the pure crystalline salts. Nevertheless, the polyanion salts can readily be purified and prepared in high purity, as described in the examples. The polyanion salts can also be prepared as mixed salts with monovalent anions such as, for example, hydrochloride, thus

1. achieving a stabilization of the inorganic salts such as, for example, of the hydrochloride,
2. achieving a higher content of active ingredient in the total composition,
3. the solubility decreasing only slightly while the substantially amorphous nature is retained.

The prepared polyanion salts comprise only few free $H^+$-ions in comparison to the clopidogrel hydrogen sulphate salt ($HSO_4^-$), so that the acidity is distinctly less and harmful side effects on excipients or the body can be avoided.

It has surprisingly been found that the desired clopidogrel-polyanion salts can be easily precipitated when solutions of freely soluble salts of clopidogrel are added to aqueous polyanion solutions in the low pH range. The precipitates can easily be removed by centrifugation or filtration. The freely soluble clopidogrel salts used as starting material preferably have inorganic anions, like clopidogrel hydrogen sulphate, the hydrobromide or the particularly readily soluble hydrochloride. An additional or optional freeze drying step with an optional subsequent removal, for instance by washing, of the substituted anions can be performed.

These starting materials can also be prepared *in situ* from free clopidogrel base before the reaction with the polyanion, in particular by the following 3 methods:

a) Dissolving the resinous clopidogrel base in methanol, reacting with an HCl solution and removing the methanol by evaporation.
b) Dissolving the resinous clopidogrel base in HCl with dispersion of-the clopidogrel drops
c) Extraction of the clopidogrel base into a water-immiscible organic solvent such as, for example, chloroform, diethyl ether or ethyl acetate and subsequent shaking with a solution of the appropriate polyanion in hydrochloric acid.

It has been found that, in contrast to the methods of preparation with monovalent anions, instead of equimolar ratios between clopidogrel and anion it is beneficial to use a distinct excess of clopidogrel. This on the one hand drastically increases the yield of precipitated salt, and on the other hand leads to a greater loading of the polymer and to a better clopidogrel/polyanion ratio.

The preparation of the clopidogrel-polyanion salts is described in some examples below and shown in the following figures.

BRIEF DESCRIPTION OF THE DRAWINGS

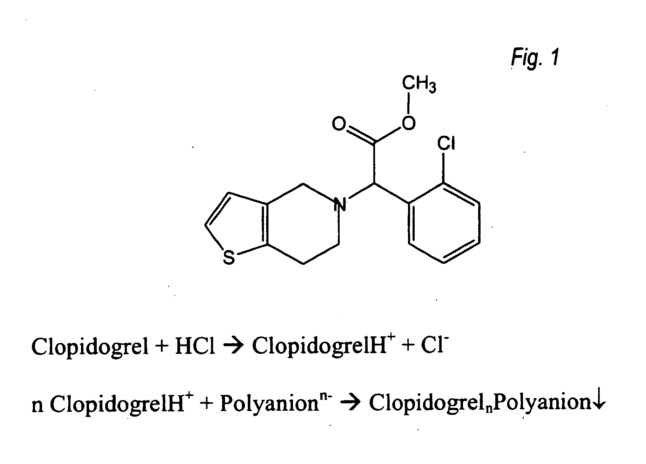

Figure 1: Structure of the active ingredient clopidogrel and reaction scheme for preparing a clopidogrel-polyanion salt

Figure 2: Absorption spectra of clopidogrel hydrochloride in water at pH 1.2 in various concentrations (solid curve (2): 0.015M and dashed curve (1): 0.0015M)

Figure 3: Plots of release of clopidogrel from the salts at 25°C in 0.05M HCl (solid line), and the maximum value at complete dissolution (dashed line)

3a) release of clopidogrel from clopidogrel-PSS
3b) release of clopidogrel from clopidogrel-carrageenan
3c) release of clopidogrel from the clopidogrel-carrageenan/HCl mixed salt

Figure 4: Powder diffractograms of the prepared clopidogrel-polyanion compounds

DETAILED DESCRIPTION OF PREFERRED EMBODIMENTS

**Analytical characterization of the sample**s

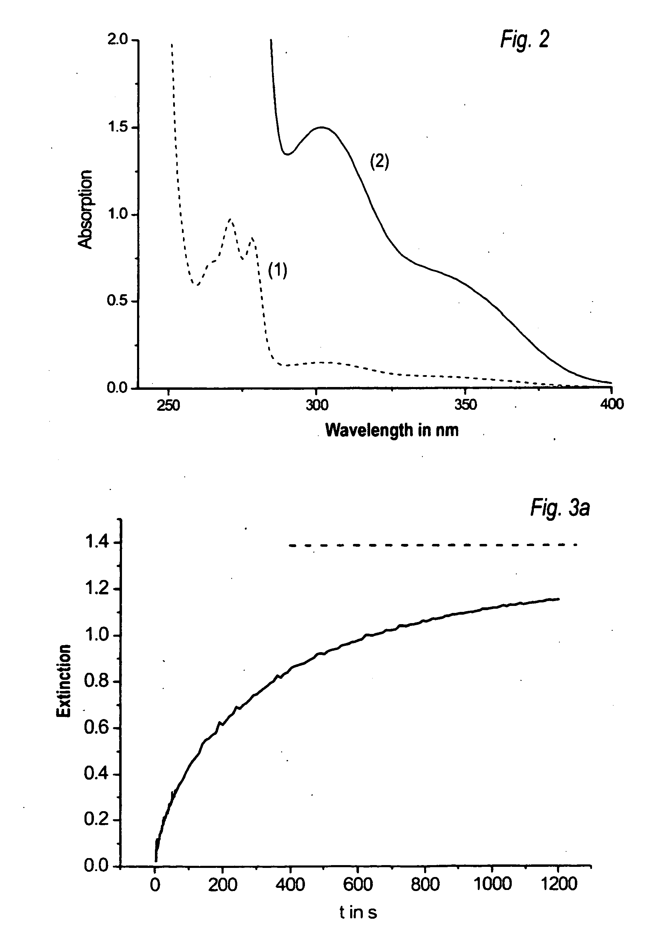

UV/Vis spectroscopy (Varian, Cary 50) was used for qualitative and quantitative characterization of the prepared compounds and provides a characteristics spectrum in the range 250-285 nm (Figure 2) for clopidogrelH$^+$ in water (the spectrum of the base in methanol differs therefrom), with characteristic UV/Vis peaks lying at

$$\lambda_{max} = 271 \text{ nm} \qquad \varepsilon = 680 \text{ L/mol} \times \text{cm}$$

$$\lambda_{max} = 278 \text{ nm} \qquad \varepsilon = 603 \text{ L/mol} \times \text{cm}$$

$$\lambda_{max} = 301 \text{ nm} \qquad \varepsilon = 105 \text{ L/mol} \times \text{cm}$$

$$\lambda_{max} = 342 \text{ nm} \qquad \varepsilon = 46 \text{ L/mol} \times \text{cm.}$$

The ratio between clopidogrel and polyanion in the prepared compounds was further ascertained by CHN analyses. The kinetics of dissolution of the clopidogrel-polyanion salts and the solubilities were determined in 0.05M HCl by UV/Vis spectroscopy. The X-ray investigations on the crystalline/amorphous ratio were carried out on powder samples with an STOE STADI P transmission diffractometer, Cu K alpha 1 radiation (1.5406 A).

### Example 1:

### Preparation of clopidogrel-PSS from clopidogrelHBr

804 mg (2 mmol) of clopidogre1HBr are dissolved in 20 mL of 0.2M HCl. 10 ml of a 0.1 monomolar solution (molarity based on monomers) of the sodium salt of polystyrenesulphonic acid (PSS) are added to this solution while stirring. The solution is cooled to 5°C while stirring, and the precipitate is then removed by centrifugation. The precipitate is washed 3 times with 1 mL of 0.05M HCl each time and then dried in vacuo.

*Yield:* theor: 505 mg, found 230 mg = 46%

*Active ingredient loading:* The active ingredient loading was analysed by dissolving 1.77 mg of product in 2 mL of 0.05M HCl (0.885 g/L) and recording the UV/Vis spectrum. The extinction at 271 nm is E = 1.628, being composed of the extinction of PSS $\varepsilon_{271}$ = 330 L/mol$\times$cm and the extinction of clopidogrel $\varepsilon_{271}$ = 680 L/mol$\times$cm to give $\varepsilon_{271}$ = 1010 L/mol$\times$cm. This results in a first approximation ($M_w$ = 505 g/mol for 1:1 product) in a loading of 58.5% m/m of clopidogrel in the product, which is somewhat below the theoretically achievable 63%. 57.9% were found in a second analysis. This means that 92% of the PSS groups are loaded with clopidogrel.

*Elemental analysis:* Elemental analysis of the starting materials and of the prepared compound yields the values listed in Table 1, using the theoretical PSS composition for calculating the compound and assuming a 1:1 loading ratio between PSS and clopidogrel in the product composition:

Table 1: Percentage distribution of the elements in the starting materials and the prepared compound *Molecular weights:* Clopidogrel hydrobromide 402 g/mol, PSS (monomer unit) 206 g/mol, clopidogrel-PSS 505.45 g/mol

| Compound | Clopidogrel hydrobromide | Clopidogrel hydrobromide | Na PSS | Clopidogrel PSS | Clopidogrel PSS |
|---|---|---|---|---|---|
| Element | % theor. | % found | % theor. | % theor. | % found |
| C | 47.71 | 47.17 | 46.60 | 56.98 | 54.64 |
| H | 4.22 | 4.365 | 3.40 | 4.75 | 5.028 |
| N | 3.48 | 3.463 | 0.00 | 2.77 | 2.773 |
| O | 7.95 | - | 23.30 | 15.83 | - |
| S | 7.95 | 7.981 | 15.53 | 12.66 | 12.31 |
| Cl | 8.81 | 8.614 | 0.00 | 7.01 | 7.4 |
| Br | 19.88 | 19.4 | 0.00 | 0.00 | - |

(continued)

| Compound | Clopidogrel hydrobromide | Clopidogrel hydrobromide | Na PSS | Clopidogrel PSS | Clopidogrel PSS |
|---|---|---|---|---|---|
| Na | 0 | - | 11.17 | 0.00 | - |
| | 100.00 | 91 | 100.00 | 100.00 | 82.15 |

The elemental analyses confirm the composition of the desired product clopidogrel-PSS. Likewise, the spectroscopic result of a somewhat lower loading is verified, because the carbon content and sulphur content are slightly lower than would be expected with 100% clopidogrel loading.

*Solubility*

The solubility of the compounds was determined by overlayering in each case 20 mg with 500 μL of 0.05M HCl and incubating at 25°C with shaking for 48 h. The precipitate was spun down thoroughly, and the supernatant was diluted in 0.05M HCl and the spectrum was recorded. The solubility L in g/L of the compound was determined from the extinction. $L_{clopiss}$= 0.0195 mol/L = 9.9 g/L

*Rate of dissolution*

The kinetics of dissolution were measured in 0.05M HCl solution (pH 1.3) at 25°C. For this purpose, a defined amount of the clopidogrel salt which had been powdered in a mortar was weighed into a 4 mL quartz cuvette and placed with a magnetic stirrer in the UV/Vis spectrometer. At time to = 0s, 2 mL of 0.05M HCl were added, and the increase in extinction at 271 nm was followed. The extinction when dissolution was complete at $t_\infty$ was determined after stirring for 20 h and briefly heating the sample at 50°C. Figure 3a shows the kinetics of dissolution and the value when dissolution is complete (dashed line). Release after 20 min at 25°C is 83% for the clopidogrel-PSS compound.

## Example 2

### Preparation of clopidogrel-carrageenan from clopidogrel base (method (a), see above)

6.42 g of clopidogrel (20 mmol) are dissolved in 100 mL of methanol at room temperature. 200 mL of 0.2M HCl (40 mmol) are added thereto. The clear solution was concentrated to about 120 mL in a rotary evaporator and made up again to 150 mL with water. A clear 0.133M clopidogrel hydrochloride solution with a pH of 0.67 is obtained. 90 mL of this solution (12 mmol) were added to 60 mL of a 0.1M solution (6 mmol) of λ-carrageenan of type IV (Sigma-Aldrich) while stirring vigorously at room temperature. The solution is cooled while stirring to 5°C, and the precipitate is filtered off. The precipitate is washed 3 times by being resuspended each time in 24 mL of 0.05M HCl and filtered. The precipitate was then dried in vacuo.

*Yield:* theor: 3324 mg, found 2042 mg = 61.4%
*Active ingredient loading:* The active ingredient loading was determined in duplicate in analogy to Example 1 and resulted in 57.26 and 57.19% respectively, i.e. 98% of the theoretical loading of 57.9%.

*Elemental analysis:* Elemental analysis of the starting materials and of the prepared compound results in the values listed in Table 2. The values measured for carrageenan differ distinctly from the values resulting from the formula. The measured carrageenan composition was used to calculate the theoretical values for the clopidogrel-carrageenan salt. The product composition was assumed to be a 1:1 charge ratio between carrageenan and clopidogrel.

Table 2: Percentage distribution of the elements in the starting materials and the prepared compound *Molecular weights:* Clopidogrel base 321 g/mol, carrageenan (monomer unit) 256 g/mol, clopidogrel-carrageenan 554 g/mol

| Compound Element | Clopidogrel base % theor. | Carrageenan 2 sulphate groups per galactopyranose dimer % theor. | Carrageenan 3 sulphate groups per galactopyranose dimer, % theor. | Carrageenan % measured | Clopidogrel carra % theor. | Clopidogrel carra % found |
|---|---|---|---|---|---|---|
| C | 47.71 | 28.24 | 22.86 | 22.28 | 44.46809 | 42.12 |
| H | 4.22 | 3.14 | 2.70 | 3.799 | 4.787874 | 4.796 |
| N | 3.48 | 0.00 | 0.00 | 0.145 | 2.560156 | 2.669 |
| O | 7.95 | 47.06 | 48.25 | - | - | - |
| S | 7.95 | 12.55 | 15.24 | 11.74 | 11.14950 | 11.75 |
| C1 | 8.81 | 0.00 | 0.00 | 0.3 | 6.291723 | 8.23 |
| Br | 19.88 | 0.00 | 0.00 | - | 0 | - |
| Na | 0 | 9.02 | 10.95 | - | 0 | - |
| Total | 100.00 | 100.00 | 100.00 | 38.26 | 64.37 | 69.6 |

The elemental analyses differ somewhat from the expected composition, possibly explicable by the non-uniform composition of the natural product carrageenan.

*Solubility*

The solubility was determined in analogy to Example 1 and is in 0.05M HCl at 25°C
$L_{clopicarra}$ = 0.0634 mol/L = 35.1 g/L

*Rate of dissolution*

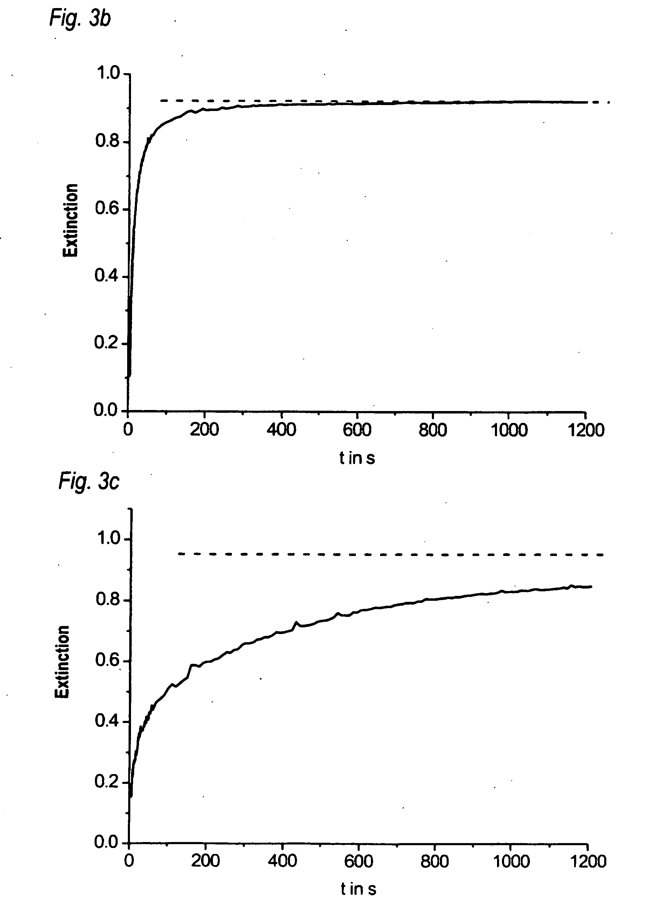

The kinetics of dissolution were determined in analogy to Example 1. Figure 3b shows the kinetics of dissolution and the value when dissolution is complete (dashed line). Release after 20 min at 25°C is 99% for the clopidogrel-carrageenan compound. The product dissolves very rapidly and dissolution is virtually 100% complete after only a few minutes.

## Example 3

**Preparation of a clopidogral-carrageenan/HCl mixed salt from clopidogrel base (method (b), see above)**

3.21 g of clopidogrel base (10 mmol) are dissolved in 50 mL of 2M HCl (100 mmol) by vigorous stirring and ultrasound. This is possible, in contrast to HCl of low concentration, within less than one minute. The solution is then adjusted to a pH of about 0 with 4 mL of 10M NaOH (90 mmol) and, while stirring vigorously, added to 100 mL of 0.05M aqueous λ-carrageenan solution (5 mmol). The pH is raised to pH 1 by adding 10M NaOH, and the solution is cooled to 5°C while stirring. The precipitate is spun down, washed 3 times with 12 mL of 0.05M HCl each time and then dried in vacuo.
*Yield:* theor: 2770 mg, found 1620 mg = 58.5%
*Active ingredient loading:* The active ingredient loading was determined in duplicate in analogy to Example 1 and the duplicate determination resulted in 72.26 and 74.1%, respectively, i.e. 126% of the theoretical value for the pure clopidogrel-carrageenan salt of 57.9% loading. The difference results from the formation of a mixed salt with clopidogrel hydrochloride as shown by the elemental analyses hereinafter. Pure clopidogrel hydrochloride could not be isolated on its own from aqueous solution and is described in the literature as being very hygroscopic. Calculation of the ratio between carrageenan anion and chloride anion from the loading (100% carrageenan salt = 57.9% loading; 100% chloride salt = 89.9% loading) results in, a carrageenan:chloride ratio of 1.13.

*Elemental analysis:* Elemental analysis of the prepared compound provided the proof of a mixed salt. The carrageenan composition determined by the elemental analysis was used to calculate the theoretical values for the clopidogrel-carrageenan salt. The product composition was assumed to be a 1:1 charge ratio between carrageenan and clopidogrel (Table 3).

Table 3: Percentage distribution of the elements in the prepared compound

| Compound | Clopidogrel carra | Clopidogrel carra |
|---|---|---|
| Element | % theor. | % found |
| C | 44.46809 | 40.25 |
| H | 4.787874 | 4.381 |
| N | 2.560156 | 2.402 |
| O | - | - |
| S | 11.14950 | 11.4 |
| Cl | 6.291723 | 11.11 |
| Br | 0 | - |
| Na | 0 | - |
| Total | 64.37 | 69.54 |

The elemental analyses differ distinctly from the expected composition, resulting from the formation of the chloride mixed salt. In particular the high value for chlorine and the ratio between the carbon content and the chlorine content confirm the formation of the mixed salt, the latter specifically agreeing well with the ratio between carrageenan anion and chloride anion derived from the loading.

*Solubility*

The solubility was determined in analogy to Example 1. In 0.05M HCl at 25°C it is $L_{clopicarra/HCl}$ = 0.026 mol/L = 14.4 g/L.
This is distinctly lower than the solubility of the pure single salts, thus explaining the preferential formation of this mixed salt at low pH.

*Rate of dissolution*

The kinetics of dissolution were determined in analogy to Example 1. Figure 3c shows the kinetics of dissolution and the value when dissolution is complete (dashed line). The clopidogrel-carrageenan/HCl mixed salt dissolves substantially less well than the pure carrageenan salt. Release after 20 min at 25°C is only 89.5%.

### Example 4:

### Preparation of clopidogrel-dextran sulphate from clopidogrel hydrogen sulphate (method c)

0.418 g of clopidogrel hydrogen sulphate (1 mmol) is dissolved in 30 mL of water and covered in a separating funnel with a layer of a mixture of 50% ethyl acetate and 50% diethyl ether. 3.5 mL of 10M NaOH are added thereto and shaken for a lengthy period. After the initially cloudy aqueous phase has become substantially clear, it is separated off. The organic phase is washed once with 20 mL of water. Then 5 mL of a 0.1M dextran sulphate solution and 5 mL of a 0.3M HCl are added and shaken for a lengthy period. A white precipitate separates out in the aqueous phase. After removal of the organic phase, the precipitate is spun down, washed 3 times with 0.05M HCl and then dried in vacuo.
*Molecular weight:* $M_w$ = 617 g/mol
*Yield:* theor: 308.5 mg, found 212 mg = 68.7%
*Active ingredient loading:* The active ingredient loading was determined in duplicate in analogy to Example 1, and the duplicate determination resulted in 59.2 and 60.7%, respectively. This is somewhat more than would have been expected from the sulphate content indicated by the manufacturer (Sigma). Only the solubilities and the percentage clopidogrel content were determined for this and the following compounds.

*Solubility*

The solubility was determined in analogy to Example 1. In 0.05M HCl at 25°C it is $L_{clopidextran\ sulphate}$ = 0.019 mol/L = 11.7 g/L. This is distinctly lower than the solubility of the pure single salts, thus explaining the preferential formation of this mixed salt at low pH.

**Example 5:**

**Preparation of clopidogrel-heparin from clopidogrel base (method a)**

The salt ($M_w$ = 584 g/mol) was prepared in analogy to Example 2 in a smaller batch. The *yield* was 22%, the *active ingredient loading* was determined to be 53.4%, almost corresponding to the theoretical value (54.9%). The *solubility* in 0.05M HCl is $L_{clopiheparin}$ = 0.084. mol/L = 49.1 g/L.

**Example 6:**

**Preparation of clopidogrel-chondroitin sulphate from clopidogrel base (method a)**

The salt was ($M_w$ = 530 g/mol) prepared in analogy to , Example 2 in a smaller batch. However, no precipitate separated out in this case. The solution was therefore freeze dried and the NaCl was removed from the resulting solid by washing 3 times. The *yield* was 15%, the *active ingredient loading* was determined to be 51.7% (theoretical 60.6%). The low active ingredient loading is perhaps explained by the extremely high *solubility* of $L_{clopichondroitin}$ sulphate = 0.45 mol/L = 238 g/L.

**Example 7:**

**Preparation of clopidogrel-polyvinyl phosphate from clopidogrel base (method a)**

The salt ($M_w$ = 443 g/mol) was prepared in analogy to Example 6. A gelatinous composition was obtained, but a powder also resulted after freeze drying. The *yield* was 35%, the *active ingredient loading* was determined to be 73.2% (theoretical 72.5% for one clopidogrel per phosphate group). The *solubility* is $L_{clopipolyvinyl\ phosphate}$ = 0-22 mol/L = 97 g/L, with insoluble particles remaining even at high dilution.

**Example 8:**

**Preparation of clopidogrel-polyvinylsulphonate from clopidogrel base (method a)**

The salt ($M_w$ = 428 g/mol) was prepared in analogy to Example 6. The *yield* was 19%, the active *ingredient loading* was determined to be 48% (theoretical 75%). The low active ingredient loading is perhaps explained by the extremely high *solubility* of the salt with $L_{clopipolyvinylsulphonate}$ = 0.4 mol/L = 171 g/L and possibly by the low hydrophobicity of the polyanion.

**Analysis of the crystallinity of the compounds**

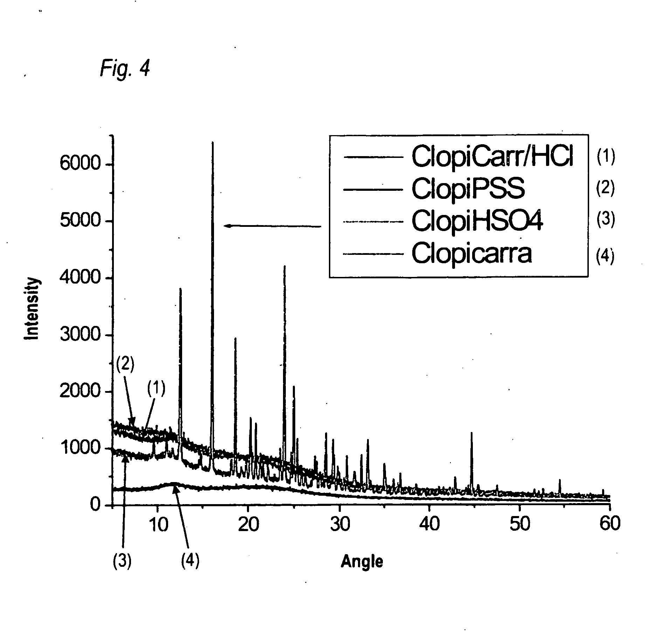

Measurements were carried out in an X-ray powder diffractometer on the samples from Examples 1-3, clopidogrel-PSS, clopidogrel-carrageenan, clopidogrel-carrageenan/HCl and, for comparison, clopidogrel hydrogen sulphate. Figure 4 shows the corresponding spectra. Whereas clopidogrel hydrogen sulphate (green; curve (3)) shows a substantially crystalline structure, the structure to be found with all the prepared products is substantially amorphous. There are still slight gradations between the clopidogrel-PSS (red in the figure; curve (2)) with a completely amorphous nature and the clopidogrel-carrageenan (curve (4)) with a quite weak structure and the clopidogrel-carrageenan/HCl (curve (1)) with a somewhat more pronounced structure, but still substantially amorphous.

**Claims**

**1.** Salts of the active ingredient clopidogrel with polyanions, wherein the salts are substantially or predominantly in amorphous form, and the polyanions have at least 4 negative charges, but preferably more than 7 and very particularly preferably more than 10 charges.

2. Salts according to Claim 1 of the active ingredient clopidogrel with organic polyanions which have as charge carriers sulphate or sulphonate or phosphate groups.

3. Salts according to Claim 1 or 2, wherein the polyanions include all types of carrageenans, chondroitin sulphate, alginate sulphate, dextran sulphate, sulphoethylcellulose and heparin.

4. Salts according to any of Claims 1-3, wherein the polyanions are synthetic polymers with preferably sulphate, sulphonate, phosphate or phosphonate groups, such as, for example, comprise polystyrenesulphonate, polyanetholesulphonate, polyvinyl sulphate, polyvinyl phosphate.

5. Salts according to any of Claims 1-3 with the polyanions heparin or DNA, which may serve as combination products.

6. Mixed salts which consist of a salt according to Claim 1-5 and monovalent clopidogrel salts, in which the polyanion salt content is at least 10%, preferably more than 30%.

7. Method for preparing a salt according to Claims 1-6, where the salt is precipitated from a predominantly aqueous solution of a freely soluble clopidogrel salt with an aqueous solution of the polyanion.

8. Use of a salt according to Claims 1-6 for manufacturing a pharmaceutical formulation.

9. Pharmaceutical formulation comprising a salt according to any of Claims 1-6.


**Patentansprüche**

1. Salze des Wirkstoffes Clopidogrel mit Polyanionen, wobei die Salze im Wesentlichen oder überwiegend in amorpher Form sind und die Polyanionen mindestens 4 negative Ladungen, bevorzugt jedoch mehr als 7 und ganz besonders bevorzugt mehr als 10 Ladungen haben.

2. Salze nach Anspruch 1 des Wirkstoffes Clopidogrel mit organischen Polyanionen, die als Ladungsträger Sulfat- oder Sulfonat- oder Phosphatgruppen haben.

3. Salze nach Anspruch 1 oder 2, wobei die Polyanionen alle Typen von Carrageenanen, Chondroitinsulfat, Alginatsulfat, Dextransulfat, Sulfoethylcellulose und Heparin umfassen.

4. Salze nach einem der Ansprüche 1-3, wobei die Polyanionen synthetische Polymere mit bevorzugt Sulfat-, Sulfonat-, Phosphat- oder Phosponatgruppen sind, wie z.B. Polystyrolsulfonat, Polyanetholsulfonat, Polyvinylsulfat, Polyvinylphosphat enthalten.

5. Salze nach einem der Ansprüche 1-3 mit den Polyanionen Heparin oder DNA, die als Kombinationsprodukte dienen können.

6. Mischsalze, die aus einem Salz nach Anspruch 1-5 und monovalenten Clopidogrelsalzen bestehen, in denen der Polyanionensalzanteil mindestens 10%, bevorzugt mehr als 30% beträgt.

7. Verfahren zur Herstellung eines Salzes nach den Ansprüchen 1-6, wobei das Salz aus einer überwiegend wässrigen Lösung eines leicht löslichen Clopidogrelsalzes mit einer wässrigen Lösung des Polyanions ausgefällt wird.

8. Verwendung eines Salzes nach den Ansprüchen 1-6 zur Herstellung einer pharmazeutischen Formulierung.

9. Pharmazeutische Formulierung, umfassend ein Salz nach einem der Ansprüche 1-6.

**Revendications**

1. Sels du principe actif clopidogrel avec des polyanions, lesquels sels sont sensiblement ou essentiellement sous forme amorphe, et les polyanions possèdent au moins 4 charges négatives, mais de préférence plus de 7 et encore plus préférablement plus de 10 charges.

2. Sels selon la revendication 1 du principe actif clopidogrel avec des polyanions organiques dont les porteurs de charge sont les groupes sulfate, sulfonate ou phosphate.

3. Sels selon la revendication 1 ou 2, dans lesquels les polyanions comprennent tous les types de carraghénines, le sulfate de chondroïtine, le sulfate d'alginate, le sulfate de dextrane, la sulfoéthylcellulose et l'héparine.

4. Sels selon l'une quelconque des revendications 1 à 3, dans lesquels les polyanions sont des polymères synthétiques ayant de préférence des groupes sulfate, sulfonate, phsophate ou phosphonate, notamment, par exemple, comprennent du sulfonate de polystyrène, du sulfonate de polyanéthole, du sulfate de polyvinyle ou du phosphate de polyvinyle.

5. Sels selon l'une quelconque des revendications 1 à 3, dont les polyanions héparine ou ADN, peuvent servir de produits de combinaison.

6. Sels mélangés comprenant un sel selon les revendications 1 à 5 et des sels monovalents de clopidogrel, dans lesquels la teneur en sel du polyanion est d'au moins 10 %, de préférence de plus de 30 %.

7. Procédé de préparation d'un sel selon les revendications 1 à 6, dans lequel le sel est précipité à partir d'une solution essentiellement aqueuse d'un sel de clopidogrel librement soluble avec une solution aqueuse du polyanion.

8. Utilisation d'un sel selon les revendications 1 à 6 pour fabriquer une formulation pharmaceutique.

9. Formulation pharmaceutique comprenant un sel selon l'une quelconque des revendications 1 à 6.

*Fig. 1*

Clopidogrel + HCl → ClopidogrelH$^+$ + Cl$^-$

n ClopidogrelH$^+$ + Polyanion$^{n-}$ → Clopidogrel$_n$Polyanion↓

Fig. 2

Fig. 3a

Fig. 3b

Fig. 3c

*Fig. 4*

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 0099802 A
- US 4529596 A
- EP 0281459 A
- EP 1480985 B1
- WO 2004072085 A2

- EP 1415993 A1
- US 6284277 B
- WO 2004026879 A
- EP 191133 A
- EP 211268 A